# EUROPEAN PATENT SPECIFICATION

(11) **EP 4 101 524 B1**
(45) Date of publication and mention of the grant of the patent: **03.09.2025**
(21) Application number: 22177857.4
(22) Date of filing: 08.06.2022
(51) Int. Cl.: B01F 21/00, B01F 23/00, B67D 1/04, B67D 1/00, A61K 33/00

(54) **BIPHASIC MIXTURE OF HYDROGEN AND CARBON DIOXIDE**
ZWEIPHASENGEMISCH AUS WASSERSTOFF UND KOHLENDIOXID
MÉLANGE BIPHASIQUE D'HYDROGÈNE ET DE DIOXYDE DE CARBONE

(30) Priority: 08.06.2021 IT 202100014912
(43) Date of publication of application: 14.12.2022
(73) Proprietor: Societa' Italiana Acetilene & Derivati S.I.A.D. S.p.A. in breve S.I.A.D. S.p.A., 24126 Bergamo (IT)
(72) Inventor: BISSOLOTTI, Giorgio, 24126 BERGAMO (BG) (IT); TURRA, Fabrizio, 24126 BERGAMO (BG) (IT); PESCALI, Piergiorgio, 24126 BERGAMO (BG) (IT)
(74) Representative: Bianchetti & Minoja with Trevisan & Cuonzo IPS SRL

(56) References cited:
- EP-A1- 3 184 164
- EP-A1- 3 689 356
- US-A1- 2019 047 729

## Description

The present invention relates to a biphasic mixture of hydrogen and carbon dioxide for use in water or beverage carbonation devices.

### Introduction

The beneficial properties of hydrogen in medicine and for sports performances are reported in numerous scientific articles.

Tissue loss deriving from inflammatory processes and/or intensive physical exercise is characterized by oxidative stress due to an increase in reactive oxygen species (ROS), hydrogen peroxide (H₂O₂) and superoxide anion (O₂-; • OH), ONOO- [1], molecules that interfere with normal cell homeostasis, causing a reduction in the fundamental cell survival signals and inducing apoptosis. Recent tests have demonstrated that H₂ is a potent antioxidant, anti-apoptotic and anti-inflammatory agent, which can therefore have potential medical applications. Hydrogen allows a scavenging action, and counteracts the effects of radicals [2,3]. Recent tests have demonstrated the pleiotropic therapeutic effects of molecular H₂ in a variety of animal disease models and clinical practice [3,4,5]. Hydrogen has elicited improvements in patients suffering from diabetes, metabolic and cholesterol regulation syndromes [3,8-10]. It has also proved preventive and curative of skin damage caused by solar radiation or other events causing skin degeneration [11-14], and it improves the quality of life of patients undergoing radiotherapy [3,15] or suffering from major depressive disorder [16]. Hydrogen has exhibited neuroprotective properties in murine models of neurodegenerative disorders [17,18,19]. Recent studies have demonstrated that intake of hydrogenated water improves resistance to muscle fatigue in sportsmen and women, improves peak oxygenation, and induces faster physical recovery due to a significant increase in ATP; NAD + / NADPH and a simultaneous reduction in waste metabolites and ROS [3,20-24]. The importance that hydrogen is acquiring is confirmed by the large number of clinical trials in progress [25].

Hydrogen can be inhaled as mixture of gases or taken dissolved in drinks. It can be dissolved by bubbling hydrogen through the liquid to be saturated or by mixing the gas with the liquid to be saturated using various devices.

The inflammability of H₂ precludes the use of many of the current carbonation devices. One solution to this problem is to dilute the hydrogen with inert gases so that its concentration falls below the limits that classify the mixture as inflammable. The most commonly used of said gases is carbon dioxide (CO₂), which is already widely used in the field of beverages and mineral waters.

One obstacle to the use of mixtures of carbon dioxide and hydrogen is the fact that CO₂ is a liquefied gas under pressure, so that a homogeneous mixture consisting of H₂ and CO₂ can be prepared up to a pressure equal to the vapor pressure of CO₂ for a given temperature; at higher pressures the CO₂ would start to liquefy, forming two phases in the container. The maximum pressures required to obtain a mixture wherein H₂ and CO₂ are perfectly mixed (Mixture A, homogeneous composition) are about 35 bars at 0°C and 26.5 bars at -10°C, the temperatures generally used as the basis for calculation in the mixture preparation field.

In view of said maximum usable pressures, to obtain a homogeneous mixture it can be calculated that a 1-litre container can contain a CO₂ and H₂ mixture containing a maximum of 71 g of CO₂ at -10°C, or 97.7 g at 0°C. If a larger mass of CO₂ is introduced, the gas in the container starts to liquefy, thus forming a liquid phase and a gas phase.

The small amount of mixture that can be introduced per unit of volume of the container is a major obstacle to economical use of said mixture. For example, the usual gas cartridges employed for domestic water carbonation devices, which currently contain about 400 g of CO₂, could only contain about 30-35 g of CO₂- H₂ mixture, assuming that the percentage concentration of hydrogen is modest, otherwise the content would be even lower.

The economic benefit which would result if it were possible to have an H₂ and CO₂ mixture in liquid phase is therefore evident, because the content per unit of volume would be similar to that of liquefied CO₂ alone.

However, hydrogen is distributed differently in the two phases, and the solubility of hydrogen in the liquid phase of CO₂ is unknown.

### Description of the invention

It has now been discovered that if the formation of two phases, liquid and gas, is possible, a given amount of H₂-CO₂ mixture can be introduced into a container so that there is enough hydrogen in the mixture extracted from the liquid phase to be used to produce drinks with dissolved hydrogen. The mixture can thus also be prevented from reaching hydrogen concentrations higher than those which, according to current standards, would cause the mixture extracted from the container to become inflammable. If the H₂-CO₂ mixture is prepared under biphasic conditions, the maximum amount of CO₂ allowed by the standards can be introduced into the container. The use of CO₂ in liquid phase allows a maximum amount of CO₂ per unit of volume corresponding to the current legislative limit to be introduced into the container, i.e. 750 g per geometric liter of the container. The amount of CO₂, and therefore of the mixture, which can be stored in the container per unit of volume is 8-10 times that storable in gas phase, a difference which is extremely important to ensure economical use (preparation, transport, number of containers) of the H₂-CO₂ mixture.

The invention therefore relates to biphasic mixtures of hydrogen and carbon dioxide for use to carbonate water or other beverages. Such a generic hydrogen-based composition is disclosed in EP 3 689 356.

"Container" here means a gas cylinder or any container which can withstand CO₂ pressure and complies with the standards governing the industry.

The containers can be of small size, such as cartridges measuring a few cm³, medium size, such as canisters or cylinders ranging from a volume of 100 cm³ to 50 liters, or large size, e.g. ranging from 50 liters to 250 m³. Conventional cryogenic containers able to contain the mixture at a minimum temperature of -78°C can also be used.

However, the storage temperature of the mixture is not a constraint, because it only influences the amount of hydrogen that can be dissolved in the liquid phase of CO₂, and the partition coefficient between the liquid phase and the gas phase.

The partition coefficient of H₂ in the gas and liquid phases of CO₂ has been determined experimentally using two types of container: cryogenic containers and cylinders.

It was found that by using H₂ concentrations of 2% mol/mol, 4% mol/mol and 8% mol/mol, said concentrations being calculated on the entire mass of CO₂ present in the container, the hydrogen concentration at 20°C present in the liquid phase is about 4.5 times less than that present in the gas phase.

For example, using a hydrogen concentration of 2% mol/mol in CO₂, having a volume occupied 50% by gas and 50% by liquid, the concentration in the liquid phase was about 0.9% mol/mol and fell to about 0.3% during use, at the final stage still in the presence of the liquid phase.

The same test, conducted with concentrations of 4% and 8% mol/mol of H₂ in CO₂, confirmed the quasi-proportionality of the concentration of dissolved H₂, amounting to about 2% and 3.5% respectively.

The same proportion is also maintained in the gas phase, when the liquid phase is exhausted.

Table 1 shows the values obtained in the experimental tests by way of example.

**Table 1**

| H₂ concentration to total CO₂ present in container. Tests at about 20°C: initial volume of liquid phase/gas phase 50%/50%. | 2% mol/mol | 4% mol/mol | 8% mol/mol |
|---|---|---|---|
| Initial concentration in liquid phase of CO₂ | 0.90 - 0.97 | 1.87 - 1.98 | 3.50 - 3.59 |
| Final concentration in liquid phase of CO₂ | 0.3 - 0.35 | 0.86 - 0.89 | 1.65 - 1.80 |
| Concentration in gas phase when liquid phase is exhausted | 4.71 - 4.94 | 9.06 - 9.17 | 19.2 - 19.4 |

The hydrogen concentration in the gas phase, before extraction from the liquid phase, amounted to 6% mol/mol, 11.5% mol/mol and 12.5% mol/mol for the mixtures with 2% mol/mol, 4% mol/mol and 8% mol/mol respectively. The variability of the results is due to experimental errors.

It is therefore possible to obtain a sufficiently high hydrogen concentration in the liquid phase to ensure that when the mixture is dissolved in a liquid, the maximum solubility of the hydrogen is reached before or at the same time as the maximum solubility of the CO₂ in water.

The solubility of CO₂ in water is 1.7 g/l (at 20°C), while that of hydrogen is 1.6 mg/l; the solubility ratio between the two is therefore about 1000.

It is therefore important for the H₂-CO₂ mixture to be consistent with said ratio, or for its ratio to be even more favorable to H₂.

Said ratio being known, it can be deduced that a useful H₂ concentration in the liquid phase of CO₂ is 0.1% and, as the partition between the gas phase and the liquid phase found experimentally is known, it can be deduced that the initial mixture must be prepared at a concentration of about 0.20% mol/mol of H₂. Again on the basis of the experimental data, knowing that the H₂ concentration in the liquid phase falls by about 2.5-3 times during extraction, the concentration of the initial dose must amount to about 0.6% mol/mol of H₂ in CO₂: in fact, the concentration of 0.1% must be obtained at the end of extraction of the liquid phase.

Thus, knowing the partition values specified above, which induce a concentration in the liquid phase of just under half the theoretical amount introduced into the mixture, and knowing the variation in H₂ concentration during extraction, it can be concluded that the industrially interesting concentrations have an initial H₂-CO₂ ratio of 1000/6.

This means that an H₂-CO₂ mixture can be prepared containing 0.6% mol/mol of H₂ for industrial carbonation uses, e.g. for water carbonation, with the certainty that the hydrogen will saturate the liquid before also saturating the CO₂. However, different concentrations of H₂ are not precluded, as they depend on the solubility of hydrogen and CO₂ relative to the liquid to be saturated.

The experimental tests demonstrate that it is possible to prepare a mixture in a container having an initial hydrogen concentration of 2% mol/mol, calculated on the total mass of CO₂, so as to reach the saturation concentration of hydrogen in the liquid wherein the hydrogen is dissolved, and so that even at the stage of final use, when the liquid phase of the mixture is exhausted, a pre-determined concentration will not be exceeded in the gas phase; for example, said concentration could be 8.02% mol/mol, i.e. the concentration which, if introduced into air, would produce an inflammable atmosphere as defined by current standards.

The mixture is extracted from the container by extracting the liquid phase first and then, when it is exhausted, extracting the gas phase. Extraction of the latter phase could be omitted at the user's discretion, if the user considers that the hydrogen concentration is too high, because at the end of the liquid phase the hydrogen concentration in the mixture rises sharply, thus instantly increasing its concentration. Non-use of the gas phase does not prejudice the advantage of using the mixture in liquid phase, because the amount of mixture remaining in the container is only a fraction of the 5-15% in mass of the entire amount of gas introduced into the container. The advantage of using the mixture in liquid phase still remains over seven times greater than that of using a container filled with mixture in homogeneous gas phase only.

The mixture can be extracted from the liquid phase either by extracting it from the bottom of the container (for example, in the case of a cylinder or canister, by positioning it with the valve at the bottom and extracting), or by maintaining the cylinder in the normal position, i.e. with the valve at the top, but fitting the valve with a suction pipe that extracts down to the base of the container.

This second solution is the most frequent in the state of the art regarding the use of mixtures in liquid phase, but the first solution, i.e. with the cylinder inverted and a valve without a suction pipe, is by no means precluded, especially for smaller containers.

The same extraction from liquid phase could be omitted in certain applications, and extraction of the mixture started from the gas phase. Said solution obviously has no impact on the amount of mixture usable, but has a major impact on the variability of the hydrogen concentrations extracted over time.

Table 2 below shows the variability of a mixture consisting of 2% mol/mol of H₂ in CO₂ if extraction always takes place from the gas phase.

**Table 2**

| | |
|---|---|
| H₂ concentration to total CO₂ present in container: cylinder filled so that liquid phase accounts for 50% of container volume. | 2% mol/mol |
| Hydrogen concentration present on first extraction | 6% |
| Hydrogen concentration after one-third of extraction time | 4% |
| Hydrogen concentration after two-thirds of extraction time | 0.5% |
| Hydrogen concentration at end of extraction time | 0.02% |

The solubility tables are given by way of example; they can obviously be constructed for each desired temperature and each CO₂ filling coefficient, i.e. for each degree of filling in mass per unit of volume.

### REFERENCES

1. Zhai X, Chen X, Ohta S, Sun X. Review and prospect of the biomedical effects of hydrogen. Med Gas Res. 2014 Nov 29;4(1):19. doi: 10.1186/s13618-014-0019-6. PMID: 25485090; PMCID: PMC4256831.
2. Huang L. Molecular hydrogen: a therapeutic antioxidant and beyond. Med Gas Res. 2016 Dec 30;6(4):219-222. doi: 10.4103/2045-9912.196904. PMID: 28217294; PMCID: PMC5223313.
3. Nicolson, G., de Mattos, G., Settineri, R., Costa, C., Ellithorpe, R., Rosenblatt, S., La Valle, J., Jimenez, A. and Ohta, S. (2016) Clinical Effects of Hydrogen Administration: From Animal and Human Diseases to Exercise Medicine. International Journal of Clinical Medicine, 7, 32-76. doi: 10.4236/ijcm.2016.71005.
4. Xue J, Shang G, Tanaka Y, Saihara Y, Hou L, Velasquez N, Liu W, Lu Y. Dosedependent inhibition of gastric injury by hydrogen in alkaline electrolyzed drinking water. BMC Complement Altern Med. 2014 Mar 3;14:81. doi: 10.1186/1472-6882-14-81. PMID: 24589018; PMCID: PMC3944674.
5. Ishibashi T. Molecular hydrogen: new antioxidant and anti-inflammatory therapy for rheumatoid arthritis and related diseases. Curr Pharm Des. 2013;19(35):6375-81. doi: 10.2174/13816128113199990507. PMID: 23859555; PMCID: PMC3788323.
6. Yang F, Yue R, Luo X, Liu R and Huang X (2020) Hydrogen: A Potential New Adjuvant Therapy for COVID-19 Patients. Front. Pharmacol. 11:543718. doi: 10.3389/fphar.2020.543718.
7. Wang M, Peng J, Hui J, Hou D, Li W, Yang J. Hydrogen therapy as an effective and novel adjuvant treatment against COVID-19. QJM. 2021 Feb 18;114(1):74-75. doi: 10.1093/qjmed/hcaa301. PMID: 33151329; PMCID: PMC7665751.
8. Kamimura N, Nishimaki K, Ohsawa I, Ohta S. Molecular hydrogen improves obesity and diabetes by inducing hepatic FGF21 and stimulating energy metabolism in db/db mice. Obesity (Silver Spring). 2011 Jul;19(7):1396-403. doi: 10.1038/oby.2011.6. Epub 2011 Feb 3. PMID: 21293445.
9. Iio A, Ito M, Itoh T, Terazawa R, Fujita Y, Nozawa Y, Ohsawa I, Ohno K, Ito M. Molecular hydrogen attenuates fatty acid uptake and lipid accumulation through downregulating CD36 expression in HepG2 cells. Med Gas Res. 2013 Mar 1;3(1):6. doi: 10.1186/2045-9912-3-6. PMID: 23448206; PMCID: PMC3599869.
10. Song G, Lin Q, Zhao H, Liu M, Ye F, Sun Y, Yu Y, Guo S, Jiao P, Wu Y, Ding G, Xiao Q, Qin S. Hydrogen Activates ATP-Binding Cassette Transporter A1-Dependent Efflux Ex Vivo and Improves High-Density Lipoprotein Function in Patients With Hypercholesterolemia: A Double-Blinded, Randomized, and Placebo-Controlled Trial. J Clin Endocrinol Metab. 2015 Jul;100(7):2724-33. doi: 10.1210/jc.2015-1321. Epub 2015 May 15. PMID: 25978109.
11. Asada R, Saitoh Y, Miwa N. Effects of hydrogen-rich water bath on visceral fat and skin blotch, with boiling-resistant hydrogen bubbles. Med Gas Res. 2019;9(2):68-73. doi:10.4103/2045-9912.260647.
12. Johny Bajgai, Kyu-Jae Lee*, Md. Habibur Rahman, Ailyn Fadriquela, Cheol-Su Kim. Role of Molecular Hydrogen in Skin Diseases and its Impact in Beauty. Journal Name: Current Pharmaceutical Design.
13. Ping Zhou, 1 Bing Lin,1 Peng Wang,1 Tao Pan,1 Shun Wang,1 Weisi Chen,2 Shaowen Cheng, 3 and Sha Liu1. The healing effect of hydrogen-rich water on acute radiation-induced skin injury in rats.
14. Ignacio RM, Kwak HS, Yun YU, Sajo ME, Yoon YS, Kim CS, Kim SK, Lee KJ. The Drinking Effect of Hydrogen Water on Atopic Dermatitis Induced by Dermatophagoides farinae Allergen in NC/Nga Mice. Evid Based Complement Alternat Med. 2013;2013:538673. doi: 10.1155/2013/538673. Epub 2013 Nov 20. PMID: 24348704; PMCID: PMC3852999.
15. Kang KM, Kang YN, Choi IB, Gu Y, Kawamura T, Toyoda Y, Nakao A. Effects of drinking hydrogen-rich water on the quality of life of patients treated with radiotherapy for liver tumors. Med Gas Res. 2011 Jun 7;1(1):11. doi: 10.1186/2045-9912-1-11. PMID: 22146004; PMCID: PMC3231938.
16. Zhang Y, Su WJ, Chen Y, Wu TY, Gong H, Shen XL, Wang YX, Sun XJ, Jiang CL. Effects of hydrogen-rich water on depressive-like behavior in mice. Sci Rep. 2016 Mar 30;6:23742. doi: 10.1038/srep23742. PMID: 27026206; PMCID: PMC4812321.
17. Matsumoto A, Yamafuji M, Tachibana T, Nakabeppu Y, Noda M, Nakaya H. Oral 'hydrogen water' induces neuroprotective ghrelin secretion in mice. Sci Rep. 2013 Nov 20;3:3273. doi: 10.1038/srep03273. PMID: 24253616; PMCID: PMC4070541.
18. Iketani M, Ohsawa I. Molecular Hydrogen as a Neuroprotective Agent. Curr Neuropharmacol. 2017;15(2):324-331. doi: 10.2174/1570159x14666160607205417. PMID: 27281176; PMCID: PMC5412697.
19. Hu HW, Chen ZG, Liu JG, Chen G. Role of hydrogen in traumatic brain injury: a narrative review. Med Gas Res. 2021; 11(3): 114-120.
20. Aoki K, Nakao A, Adachi T, Matsui Y, Miyakawa S. Pilot study: Effects of drinking hydrogen-rich water on muscle fatigue caused by acute exercise in elite athletes. Med Gas Res. 2012 Jul 12;2:12. doi: 10.1186/2045-9912-2-12. PMID: 22520831; PMCID: PMC3395574.
21. LeBaron TW, Laher I, Kura B, Slezak J. Hydrogen gas: from clinical medicine to an emerging ergogenic molecule for sports athletes 1. Can J Physiol Pharmacol. 2019 Sep;97(9):797-807. doi: 10.1139/cjpp-2019-0067. Epub 2019 Apr 10. PMID: 30970215.
22. Da Ponte A, Giovanelli N, Nigris D, Lazzer S. Effects of hydrogen rich water on prolonged intermittent exercise. J Sports Med Phys Fitness. 2018 May;58(5):612-621. doi: 10.23736/S0022-4707.17.06883-9. Epub 2017 Apr 26. PMID: 28474871.
23. P. Drid, T. Trivic, C. Casals, S. Trivic, M. Stojanovic, S.M. Ostojic, Is molecular hydrogen beneficial to enhance post-exercise recovery in female athletes?, Science & Sports, Volume 31, Issue 4, 2016, Pages 207-213, https://doi.org/10.1016/j.scispo.2016.04.010.
24. Hori A, Sobue S, Kurokawa R, Hirano S, Ichihara M, Hotta N. Two-week continuous supplementation of hydrogen-rich water increases peak oxygen uptake during an incremental cycling exercise test in healthy humans: a randomized, single-blinded, placebo controlled study. Med Gas Res. 2020;10(4):163-169.
25. https://clinicaltrials.gov/

## Claims

1. A biphasic mixture of 2- 4% mol/mol hydrogen and 98/96% mol/mol carbon dioxide, whereby the carbon dioxide is in both the gas phase and the liquid phase and hydrogen is partly dissolved in the liquid phase and partly in the gas phase, for use to carbonate water or beverages.

2. A beverage carbonation device comprising a container containing the biphasicmixture according to claim 1.

3. A device according to claim 3 wherein the container is a cylinder or container for liquefied cryogenic gases.

4. A device according to claim 2 wherein the container is fitted with a valve with a suction pipe extending to the bottom of the container.

5. A device according to any one of claims 2 and 3 wherein the dispensing means comprise a valve for extracting the mixture in the liquid phase and a valve for extraction in the gas phase.

6. A device according to claim 5 wherein the dispensing means comprise two opposing valves located at the top and the bottom of the container.

7. A device according to any one of claims 2 and 3 wherein the dispensing means allow extraction from the gas phase only.

8. A device according to any one of claims 2-7 for carbonating water, mineral water, wines or alcoholic beverages, or non-alcoholic beverages.

## Patentansprüche

1. Zweiphasiges Gemisch aus 2-4% Mol/Mol Wasserstoff und 98/96% Mol/Mol Kohlendioxid, wobei das Kohlendioxid sowohl in der Gasphase als auch in der flüssigen Phase vorliegt und Wasserstoff teilweise in der flüssigen Phase und teilweise in der Gasphase gelöst ist, zur Verwendung zur Karbonisierung von Wasser oder Getränken.

2. Getränkekarbonisierungsvorrichtung, umfassend einen Behälter, der das zweiphasige Gemisch nach Anspruch 1 enthält.

3. Vorrichtung nach Anspruch 3, wobei der Behälter ein Zylinder oder Behälter für verflüssigte kryogene Gase ist.

4. Vorrichtung nach Anspruch 2, wobei der Behälter mit einem Ventil mit einem Saugrohr ausgestattet ist, das sich zum Boden des Behälters erstreckt.

5. Vorrichtung nach einem der Ansprüche 2 und 3, wobei die Abgabemittel ein Ventil zum Extrahieren des Gemischs in der flüssigen Phase und ein Ventil zum Extrahieren in der Gasphase umfassen.

6. Vorrichtung nach Anspruch 5, wobei die Abgabemittel zwei gegenüberliegende Ventile umfassen, die sich an der Oberseite und der Unterseite des Behälters befinden.

7. Vorrichtung nach einem der Ansprüche 2 und 3, wobei die Abgabemittel eine Extraktion nur aus der Gasphase ermöglichen.

8. Vorrichtung nach einem der Ansprüche 2-7 zur Karbonisierung von Wasser, Mineralwasser, Weinen oder alkoholischen Getränken oder nichtalkoholischen Getränken.

## Revendications

1. Mélange biphasique de 2-4 % mol/mol d'hydrogène et de 98/96 % mol/mol de dioxyde de carbone, grâce à quoi le dioxyde de carbone est à la fois dans la phase gazeuse et dans la phase liquide, et l'hydrogène est partiellement dissous dans la phase liquide et partiellement dans la phase gazeuse, pour une utilisation pour gazéifier de l'eau ou des boissons.

2. Dispositif de gazéification de boissons comprenant un récipient contenant le mélange biphasique selon la revendication 1.

3. Dispositif selon la revendication 3, dans lequel le récipient est une bonbonne ou un contenant pour gaz cryogéniques liquéfiés.

4. Dispositif selon la revendication 2, dans lequel le récipient est équipé d'une vanne avec un tuyau d'aspiration s'étendant jusqu'au fond du récipient.

5. Dispositif selon l'une quelconque des revendications 2 et 3, dans lequel les moyens de distribution comprennent une vanne pour l'extraction du mélange dans la phase liquide et une vanne pour l'extraction dans la phase gazeuse.

6. Dispositif selon la revendication 5, dans lequel les moyens de distribution comprennent deux vannes opposées situées en haut et en bas du récipient.

7. Dispositif selon l'une quelconque des revendications 2 et 3, dans lequel les moyens de distribution permettent l'extraction à partir de la phase gazeuse uniquement.

8. Dispositif selon l'une quelconque des revendications 2-7, pour la gazéification d'eau, d'eau minérale, de vins ou de boissons alcoolisées, ou de boissons non alcoolisées.
